# EUROPEAN PATENT APPLICATION

(11) **EP 0 888 791 A1**
(43) Date of publication of application: **07.01.1999**
(21) Application number: 97304909.1
(22) Date of filing: 04.07.1997
(51) Int. Cl.: A61M 5/30, A61K 9/48

(54) **Syringe and drug capsule therefor**

(71) Applicant: PowderJect Research Limited, Oxford OX4 4GA (GB)
(72) Inventor: Potter, Charles David Ogilvy, Stardlake OX8 7SG (GB); Potter, David S., Cowes, Isle of Wight PO31 8BN (GB)
(74) Representative: Jackson, Peter Arthur

(57) **Abstract**

A syringe, of the kind in which a dose of the drug is entrained by a fluid flow for delivery, is characterised by an upstream portion which provides, or is arranged to provide, a source of fluid under pressure; a downstream nozzle portion; between the upstream and downstream portions an intermediate portion having two relatively sliding parts (34,35) providing therebetween a closed pocket (40) containing the dose; and an actuator mechanism (16,18,21) for initiating a flow of the fluid from the source whereupon the pressure of the fluid applies to one of the slidable parts a force causing the one part to slide relatively to the other part and to open the pocket thereby exposing the dose for entrainment by the fluid as it flows through the intermediate portion and to and through the nozzle.

## Description

The invention relates to a syringe for delivering a drug (which term includes genetic material) in controlled doses into body tissue for curative, prophylactic, diagnostic or other medical treatment. The syringe is of the kind having an upstream portion, which contains, or is arranged to be connected to, a source of fluid under pressure, a downstream nozzle portion, and, between the upstream and downstream portions, an intermediate portion which accommodates a drug dose to be delivered, and an actuator mechanism for initiating a flow of the fluid from the source so that the dose is entrained in the fluid flow through the intermediate portion and hence to and through the nozzle for delivery to a target site.

Needleless syringes of this kind are disclosed, for example, in our earlier international patent applications WO 94/24263 and WO 96/25190. In those disclosures the drug dose was provided in a sealed capsule having upstream and downstream rupturable diaphragms, sealed together about their edges and containing in the chamber therebetween them the dose in particulate form. Upon release of a compressed gas, the gas pressure quickly built up behind the capsule until the differential pressure across the capsule was sufficient to burst the capsule diaphragms, thus releasing through the spent capsule and nozzle a supersonic gas flow in which the particles were entrained.

Although this arrangement has proved to be very successful, it would be desirable to provide a capsule which is cheap to manufacture, from which the full dose of drug will reliably be flushed out and entrained in the gas stream, and which is sufficiently robust to avoid the production of debris.

In accordance with the present invention, a syringe, of the kind in which a dose of the drug is entrained by a fluid flow for delivery, comprises an upstream portion which provides, or is arranged to provide, a source of fluid under pressure; a downstream nozzle portion; between the upstream and downstream portions an intermediate portion having two relatively sliding parts providing therebetween a closed pocket containing the dose; and an actuator mechanism for initiating a flow of the fluid from the source whereupon the pressure of the fluid applies to one of the slidable parts a force causing the one part to slide relatively to the other part and to open the pocket thereby exposing the dose for entrainment by the fluid as it flows through the intermediate portion and to and through the nozzle.

The relatively slidable parts may be formed or assembled integrally with the intermediate portion of the syringe and the intermediate and downstream portions of the syringe may then be disposable. However, for ease of production, storage and distribution, particularly when the same basic syringe construction may be used for delivering a variety of different drugs, the two relatively slidable parts may provide a separate pre-filled and sealed capsule which, before use, is loaded into a chamber in the intermediate portion of the syringe.

The invention therefore also includes a prefilled and sealed capsule containing a dose of drug and arranged to be accommodated in the chamber of a syringe between an upstream source of fluid under pressure and a downstream nozzle portion, the capsule comprising two relatively slidable parts providing therebetween a closed pocket containing the dose, one of the slidable parts being arranged to slide, when subjected to the fluid pressure, relatively to the other part to open the pocket and expose the dose for entrainment in the fluid flowing through the chamber to the nozzle.

The two relatively slidable parts of the syringe or capsule may be simple plastic mouldings which have confronting surfaces which slide over one another. The surfaces may be coaxial cylindrical surfaces or other surfaces with confronting flat or curved planes parallel to the direction of sliding. The pocket may then be provided by a recess or recesses, such as an annular groove, in one or both of the parts, the pocket being closed on the upstream and downstream sides by the confronting surfaces. When the parts slide relatively to one another, at least one surface rides off its confronting surface, to open the pocket to the fluid flow, possibly around, but preferably through at least one of the parts. The flow path itself may be opened upon the relatively sliding movement of the two parts.

In order that the two parts do not slide relatively to one another to release the dose prematurely, either before the fluid flow has reached substantially maximum velocity, or, in the case in which the flow path is opened by the relative sliding of the parts, before the pressure has built up sufficiently that when the flow path has been opened to release the fluid, it accelerates very quickly to maximum velocity, a resistance against the initial sliding movement of the parts may be provided. This may take the form of an adequate interference fit between the two parts, or a complementary detent and/or projection which is overridden when the slidable part receives sufficient force from the fluid.

A close fit between the slidable parts may be sufficient to seal the dose-containing pocket. However, seals such as O rings may be provided between the relatively sliding parts. Also, when the capsule is a separate insert into a chamber of the syringe, an O ring or another seal may be provided between the capsule and syringe body to avoid fluid leakage past the capsule.

Although the invention is applicable to the syringe in which the dose is of any appropriate liquid or solid form or entrainment, and the fluid is a liquid or gas, the invention is particularly applicable to needleless syringes in which the fluid is a compressible gas, such as helium, and the dose of drug is in particulate form, such as heavy microparticles coated with DNA for genetic treatment, or powdered drugs or DNA for firing, eg transdermally, into tissue.

Some examples of syringes and drug capsules constructed in accordance with the present invention are illustrated in the accompanying drawings in which:
Fig. 1 is an axial section through a first example;
Figs. 2 and 3 are enlargements of an intermediate portion of the Fig. 1 example, showing the parts before and after firing;
Figs. 4 and 5 are sections similar to Figs. 2 and 3 but of a second example;
Figs. 6 and 7 are sections similar to Figs. 2 and 3 but showing a third example;
Figs. 8 and 9 are sections similar to Figs. 2 and 3 but showing a fourth example;
Figs. 10 and 11 are side elevations of the capsule of the fourth example before and after firing; and,
Fig. 12 is a plan of the capsule of the fourth example, but without a locating O-ring.

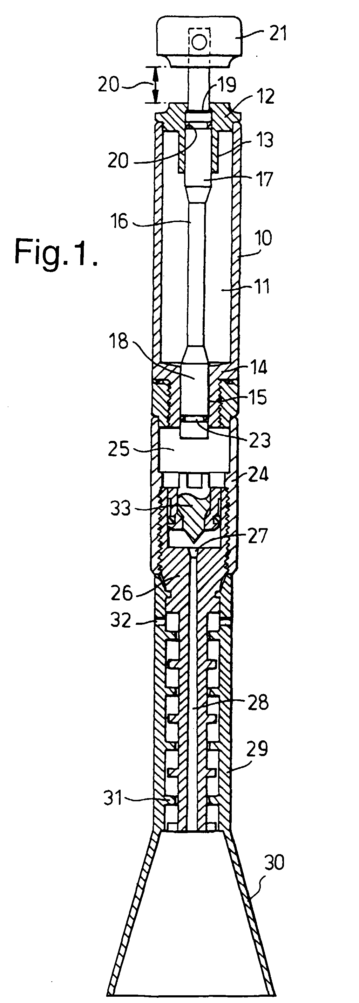

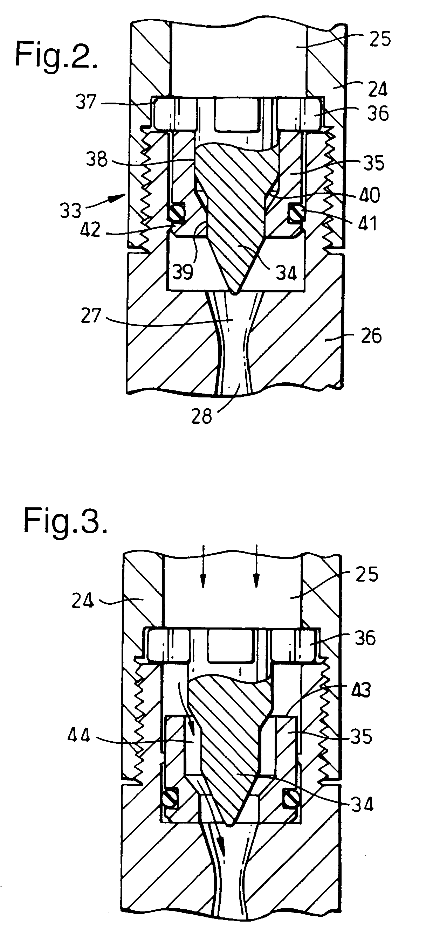

The first example illustrated in Fig. 1 is, except for the intermediate portion shown in Figs. 2 and 3, substantially identical to that of Figs. 1 to 3 of WO 94/24263. Thus it comprises an upper cylindrical barrel portion 10 containing a reservoir 11 which is prefilled with helium gas at a pressure of, typically 40-80 bar. The upper end of the barrel portion 10 is closed by an end cap 12, having a depending skirt 13. The lower end of the barrel portion 10 is closed by an integral end wall 14 formed with a depending externally screw threaded skirt 15. A plunger 16 has upper and lower cylindrical enlargements 17 and 18, which slide within the skirts 13 and 15 respectively. Upward movement of the plunger is limited by abutment of the upper end of the enlargement 17 with a shoulder 19 in the end cap 12. The plunger can be moved downwardly from this position through a stroke equivalent to the gap 20 by downward pressure on a button 21 fixed to the upper end of the plunger 16. This is conveniently performed by the operator holding the barrel 10 in the palm of his hand with his thumb overlying the button. Throughout the stroke, the enlargement 17 remains sealed to the skirt 13 by means of an O ring 22. In the raised position of the plunger, the enlargement 18 is sealed to the skirt 15 by means of an O ring 23, to seal the reservoir 11, but when the plunger is pushed downwardly, the seal 23 exits the lower end of the skirt 15 to provide a quick opening valve which releases gas from the reservoir around the clearance between the smaller diameter portion of the plunger 16 and the skirt 15.

Screwed to the bottom of the upper barrel portion 10 is a lower cylindrical barrel portion 24 containing a pressure chamber 25. Screwed into the lower end of the barrel portion 24 is a nozzle 26 having an internal passage presenting a short upstream convergent section 27 and a longer downstream divergent section 28. The nozzle 26 has fixed to it a surrounding shroud presenting a cylindrical silencer portion 29 and a divergent spacer portion 30, which extends beyond the end of the nozzle. Interdigitating baffles 31 extend radially inwardly from the cylindrical portion 29 and radially outwardly from the nozzle 26 to provide a tortuous path from within the divergent portion 30 of the shroud to a ring of vents 32. The silencer is constructed and assembled as described in WO 94/24263.

The first example differs from that of WO 94/24263 in the construction of the replaceable drug particle capsule 33, which is seen in Fig. 1 but more readily understood from Figs. 2 and 3. The capsule, which may be a prefilled and sealed unit to be inserted into a chamber defined by the parts 24,26 after unscrewing the parts 24,26, and then screwing them together again. The capsule consists essentially of two parts, a stationary core 34 and a sliding outer sleeve 35. The core 34 is formed integrally with a ring of radially extending wings 36 which are arranged to be trapped, when the parts 24 and 26 are screwed together, between a shoulder 37 on the barrel portion 24 and the upper end of the nozzle 26. The core 34 is thus held fast. The sleeve 35 has larger and smaller diameter cylindrical surfaces 38 and 39 which initially confront, as a slight interference fit, complementary cylindrical surfaces of the core 34. However, the lengths of the cylindrical surfaces are such that, initially, they provide between their transition portions, between the cylindrical surfaces of different diameter, an annular pocket 40 in which the drug particles are contained. An O ring 41, fitted in an annular groove in the sleeve 35 initially abuts an annular projection 42 on the upwardly projecting part of the nozzle 26 thereby acting to retain the parts 34 and 35 in the Fig. 2 configuration.

When the syringe is to be fired, the wider end of the spacer 30 is engaged with a patient's skin and the button 21 is depressed as previously described to release gas from the reservoir 11 into the pressure chamber 25. The pressure build up is substantially instantaneous to a value at which, acting on the annular upper face 43 of the sleeve 35, it causes the O ring 41 to override the projection 42 and the sleeve to move suddenly down to the Fig. 3 configuration, in which the gas in the pressure chamber 25 can pass, as shown by the arrows in Fig. 3, between the wings 36, and through the passage 44 which is now open as a result of the cylindrical surfaces of the sleeve 35 sliding off the complementary confronting cylindrical surfaces of the core 34. The particles thus released from the pocket 40 are swept out through the passage 44 and hence into the nozzle 26 where the accelerating gas flow approaches supersonic velocity or greater, with the drug particles entrained therein, until the particles impinge upon and penetrate the patient's skin. The shockwave reflected from the patient's skin is reflected back through the tortuous path between the baffles 31 to the vents 32 from which the gas is released with minimum noise.

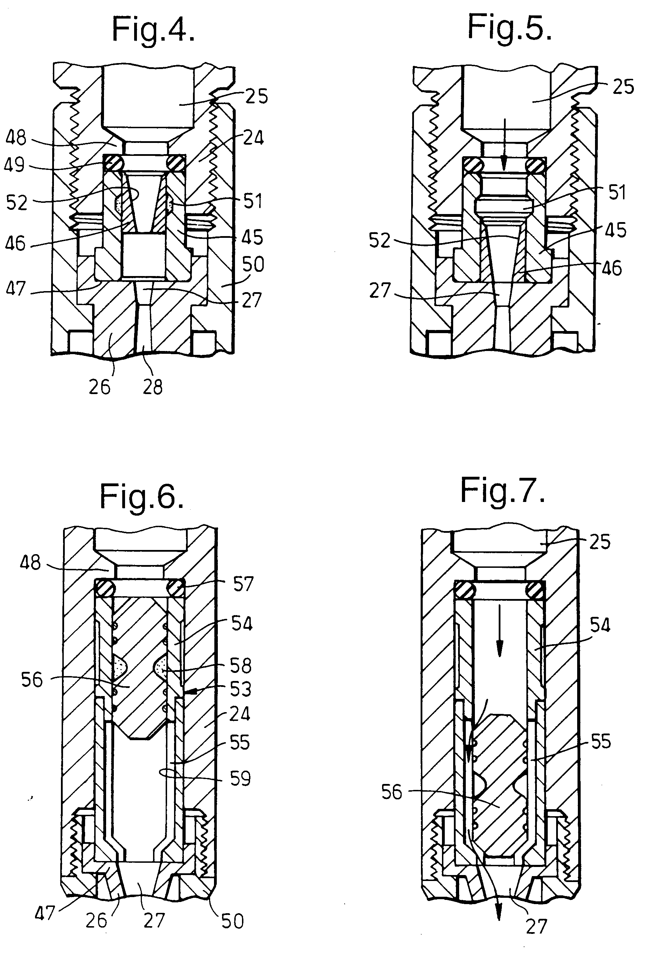

The second example illustrated in Figs. 4 and 5 has parts which are analogous in function to those of the first example and such parts are given the same reference numerals as in Figs. 1 to 3. In this case the replaceable drug-containing capsule consists of a stationary outer sleeve 45 and a slidable internal hollow core sleeve 46, the passage through which is divergently frustoconical. The outer sleeve 45 is located between a shoulder 47 at the upper end of the nozzle 26 and a shoulder 48 on the lower barrel portion 24, with an interposed O ring 49. In this case the nozzle 26 is not screwed directly to the lower barrel portion 24, but is secured to it by a gland fitting 50, which may form an upper end of the silencer and spacer shroud.

Initially the drug particles are contained in a pocket 51 formed by an annular groove in the inner surface of the outer sleeve 45, and initially closed by the outer cylindrical surface of the core 46 confronting the complementary inner cylindrical surface of the outer sleeve 45.

When the gas is released and pressure builds up almost instantaneously in the pressure chamber 25, the pressure acts on the internal surface 52 of the inner sleeve 46 and causes it suddenly to slide down to the configuration shown in Fig. 5, in which the pocket 51 is opened to the gas flow passing down from the chamber 25 to the passage 27,28 through the nozzle 26, flushing out and entraining the particles which are now released. It will be seen that the internal frustoconical surface of the inner sleeve 46 complements the upstream convergent portion 27 of the nozzle passage.

The third example, illustrated in Figs. 6 and 7, has parts bearing the same reference numerals as parts of analogous function in the first two examples. In this example the lower barrel portion 24 is bonded to the upper end of the nozzle 26, and a gland fitting 50 screws into the bottom of the lower barrel portion to locate the replaceable drug particle-containing capsule between the shoulders 47 and 48. The capsule has an outer stationary sleeve 53 with an upper generally cylindrical solid upstream portion 54 and an internally axially ribbed downstream portion 55; and an internal axially sliding core 56. An O ring 57 seals the capsule to the lower barrel portion 24. The core has an annular groove forming a pocket 58 in which the drug particles are initially contained. The pocket is sealed by upstream and downstream cylindrical external surfaces of the core confronting the cylindrical internal surface of the upstream portion 54 of the outer sleeve 53. The core 56 will be a slight interference fit within the sleeve 53.

Upon firing, the gas which is released into the pressure chamber 25 acts on the upper face of the core 56 and, when the pressure has reached sufficient value, it forces the core downwards so that the pocket 58 is then clear of the solid portion 54 but in alignment with the axially slotted portion 55 of the outer sleeve 53. This is shown in Fig. 7 and it will be seen that the gas in the chamber 25 is free to pass (as shown by the arrows in Fig. 7) down through the portion 54 of the outer sleeve, between the chamfered upper end of the core 56 and hence through the channels between the ribs 59 of the portion 55 to the nozzle passage 27. The drug particles are thus swept out and entrained in the gas flow as with the previous examples.

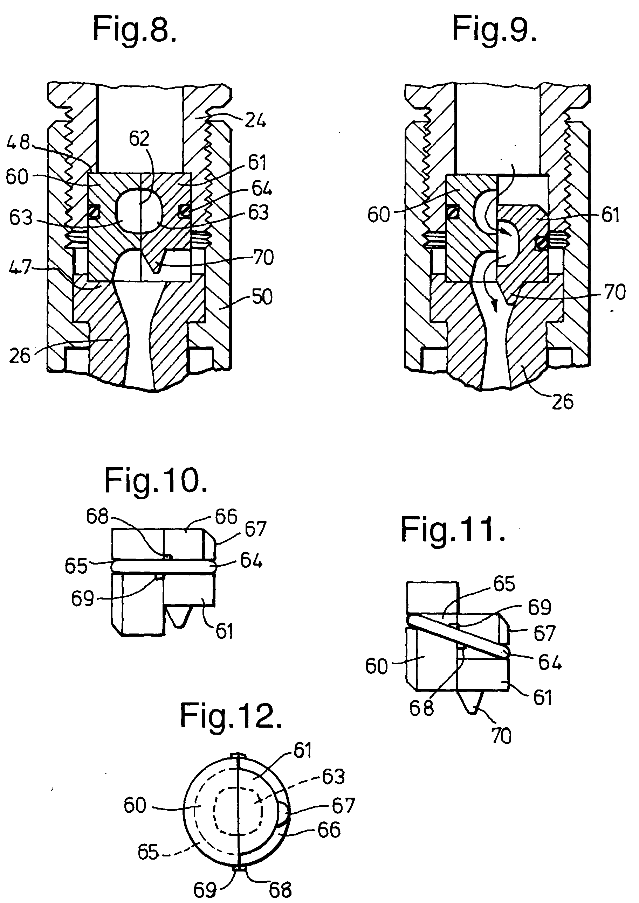

The fourth example, illustrated in Figs. 8 to 12, has parts bearing the same reference numerals as parts of analogous function in the first three examples. In this example, similarly to the second example, the replaceable capsule is located between shoulders 47 and 48 on the nozzle 26 and lower barrel portion 24, but without the need for the O ring 49. The capsule consists of two generally semi-cylindrical portions 60 and 61 which have confronting flat faces abutting one another across an axial plane 62. Both portions 60 and 61 are provided with a respective recess 63 so that in the initial configuration of the capsule before firing, the recesses oppose one another to form a sealed drug particle containing cavity. The parts 60 and 61 are initially held in this configuration by an O ring 64 as best seen in Figs. 10 to 12. The O ring also serves the function of sealing the capsule to the barrel portion 24. The O ring 64 is located in a semi-annular groove 65 which extends around the periphery of the portion 60, and in a radially recessed upper part 66 of the portion 61, beneath a projecting nib 67. In this configuration the O ring 64 lies below a small projection 68 on one or both sides of the portion 61 and above a similar small projection 69 on one or both sides of the portion 60. Thus, the portion 60 is held trapped between the shoulders 47 and 48 while the portion 61 is capable of sliding, when subjected to sufficient pressure on its upper surface, downwardly relatively to the portion 60 from the Figs. 8 and 10 position to that shown in Figs. 9 and 11. This sliding movement is initially prevented by the tension in the O ring 64 and its abutment with the projections 68 and 69. However, upon firing, when the gas pressure released from the cartridge builds up sufficiently, the force on the upper surface of the portion 61 enables the O ring 64 to override the projection 69, and the projection 68 to underride the O ring, so that the portion 61 moves suddenly downwards to the Figs. 9 and 11 position. In this position a depending part 70 of the portion 61 mates with the top of the nozzle 26 to provide a passage leading into the nozzle. As will be appreciated from Fig. 9, the previously confronting surfaces of the portions 60 and 61, have now moved out of alignment with one another, thereby opening the previously sealed drug particle-containing cavity and enabling the gas flow to pass sinuously through the capsule, as shown by the arrows and into the passageway through the nozzle 26, flushing out and entraining the particles in the gas flow.

## Claims

1. A syringe, of the kind in which a dose of a drug is entrained by a fluid flow for delivery, is characterised by an upstream portion (10) which provides, or is arranged to provide, a source of fluid under pressure; a downstream nozzle portion (26); between the upstream and downstream portions an intermediate portion having two relatively sliding parts (34,35; 45,46; 54,56) providing therebetween a closed pocket (40; 51; 58) containing the dose; and an actuator mechanism (16) for initiating a flow of the fluid from the source whereupon the pressure of the fluid applies to one of the slidable parts a force causing the one part to slide relatively to the other part and to open the pocket thereby exposing the dose for entrainment by the fluid as it flows through the intermediate portion and to and through the nozzle.

2. A prefilled and sealed capsule (33) containing a dose of drug and arranged to be accommodated in the chamber (24,26) of a syringe between an upstream source (10) of fluid under pressure and a downstream nozzle portion (26), the capsule being characterised by two relatively slidable parts (34,35; 45,46; 54,56) providing therebetween a closed pocket (40,51,58) containing the dose, one of the slidable parts being arranged to slide, when subjected to the fluid pressure, relatively to the other part to open the pocket and expose the dose for entrainment in the fluid flowing through the chamber to the nozzle.

3. A syringe according to claim 1 or a capsule according to claim 2, wherein the two relatively slidable parts have confronting surfaces (38,39) which slide over one another, the pocket being provided by at least one recess in one or both of the parts, the pocket being closed on the upstream and downstream sides by the confronting surfaces.

4. A syringe or capsule according to claim 3, in which, in order that the two parts do not slide relatively to one another to release the dose prematurely, a resistance (42) against the initial sliding movement of the parts is provided.

5. A syringe or capsule according to claim 4, in which the resistance is provided by a complementary detent and/or projection (42) which is overridden when the slidable part (35) receives sufficient force from the fluid.

6. A syringe according to any one of the preceding claims, in which the fluid is a compressible gas and the dose of drug is in particulate form.
